# EUROPEAN PATENT SPECIFICATION

(11) **EP 1 891 204 B1**
(45) Date of publication and mention of the grant of the patent: **07.04.2010**
(21) Application number: 06727061.1
(22) Date of filing: 10.05.2006
(51) Int. Cl.: C12N 5/00, C12N 5/073, C07K 16/18, C07K 16/28, G01N 33/53, G01N 33/569

(54) **METHOD OF FETAL CELL ENRICHMENT**
VERFAHREN ZUR ANREICHERUNG FÖTALER ZELLEN
PROCEDE D'ENRICHISSEMENT DE CELLULES FETALES

(30) Priority: 10.05.2005 GB 0509500
(43) Date of publication of application: 27.02.2008
(73) Proprietor: Revealcyte, London, EC2M 2TD (GB)
(72) Inventor: LEVICAR, Natasa, London W11 2JR (GB); GORDON, Myrtle, Binfield, Berkshire RG42 5QH (GB)
(74) Representative: Gardner, Rebecca Katherine
(86) International application number: PCT/GB2006/001701
(87) International publication number: WO 2006/120434

(56) References cited:
- WO-A-94/25873
- WO-A-2005/123779
- LITTLE M-T ET AL: "FREQUENCY OF FETAL CELLS IN SORTED SUBPOPULATIONS OF NUCLEATED ERYTHROID AND CD34+ HEMATOPOIETIC PROGENITOR CELLS FROM MATERNAL PERIPHERAL BLOOD" BLOOD, W.B. SAUNDERS, PHILADELPHIA, VA, US, vol. 89, no. 7, 1 April 1997 (1997-04-01), pages 2347-2358, XP001015906 ISSN: 0006-4971
- GUETTA E ET AL: "Hematopoietic progenitor cells as targets for non-invasive prenatal diagnosis: Detection of fetal CD34+ cells and assessment of post-delivery persistence in the maternal circulation." BLOOD CELLS MOLECULES AND DISEASES, vol. 30, no. 1, January 2003 (2003-01), pages 13-21, XP002390566 ISSN: 1079-9796
- COATA GIULIANA ET AL: "Prenatal diagnosis of genetic abnormalities using fetal CD34+ stem cells in maternal circulation and evidence they do not affect diagnosis in later pregnancies" STEM CELLS (MIAMISBURG), vol. 19, no. 6, 2001, pages 534-542, XP002390567 ISSN: 1066-5099
- CAMPAGNOLI CESARE ET AL: "Expandability of haemopoietic progenitors in first trimester fetal and maternal blood: implications for non-invasive prenatal diagnosis." PRENATAL DIAGNOSIS. JUN 2002, vol. 22, no. 6, June 2002 (2002-06), pages 463-469, XP002390568 ISSN: 0197-3851

## Description

The present invention relates to a method of enriching fetal cells in a maternal blood sample and to a method of obtaining an analysable sample of fetal cells. The sample obtained by the methods of the present invention is particularly useful for non-invasive fetal diagnosis and gender determination.

There is a great demand for non-invasive fetal diagnosis and gender determination. About 0.7% of all live-born infants have a congenital abnormality associated with a chromosomal defect and pre-natal diagnosis of such defects is therefore of great interest. Examples of chromosomal disorders which can be detected (if present) in fetal cells include the following Syndromes (Edwards, Patau, Downs, DiGeorge, Wolf-Hirschom, Cri du chat) and various conditions caused by microdeletions.

Current procedures for pre-natal fetal diagnosis include amniocentesis and chorionic villus sampling. Amniocentesis involves insertion of a needle through the abdomen of the pregnant female into the uterus and withdrawal of amniotic fluid from the sac surrounding the fetus. Chorionic villus sampling (CVS) is performed by inserting a catheter or needle into the placenta and removing a small tissue sample. These procedures are invasive and consequently cause discomfort to the mother and carry a risk, particularly to the fetus. The risk of fetal loss ranges from about 0.5% to 1% but may be as high as 2%. Some studies indicate that CVS may cause defects in the infant's fingers and toes. Amniocentesis is performed during or after the 15th week of pregnancy and CVS between the 10th and 12th weeks to minimise the risk for the fetus. It would be preferable to have a method which could be performed at an earlier stage of pregnancy. The cost of these procedures is also considerable. There is thus a need to develop non-invasive alternative methods. The analysis of fetal cells from peripheral maternal blood represents a non-invasive method.

A variety of fetal cells (e.g. erythroblasts, lymphocytes) enter the maternal blood stream during pregnancy in small numbers and the presence of fetal cells in maternal peripheral blood has been determined by many studies. However, the frequency of fetal cells in normal maternal peripheral blood is usually very low, ranging from 1:10⁵ to 1:10⁹. This frequency is too low to allow direct analysis of the fetal cells.

Indirect analysis can be carried out using DNA techniques such as polymerase chain reaction (PCR) amplification of fetal DNA. For example, PCR can be used to test for the presence in a maternal blood sample of a gene that is uniquely paternally inherited such as the sry gene (located on the Y chromosome) or, if the mother is rhesus negative, the rhesus gene. However, because this approach is indirect it is not suitable for all types of desired applications, e.g. it can not be used for the diagnosis of fetal chromosomal disorders such as aneuploidies in individual cells of fetal origin.

By contrast, direct methods allow the detection of a genetic disorder as well as determination of the gender in individual cells.

Direct analysis of fetal cells requires enrichment of the maternal blood sample for fetal cells to obtain an analysable sample. A variety of methods have been tried in the past.

Many groups have attempted to expand fetal cells such as fetal erythroid progenitors or fetal haemapoietic progenitors through culture (e.g. Chen et al., 1998, Manotaya et al., 2002 and Campagnoli et al. 2002). However, culture methods had little success, especially in detecting fetal cells in maternal blood before 16 weeks gestation. Furthermore, culture methods are time consuming as 2-4 weeks of incubation are required before the fetal cells can be analysed. There is also a small chance that the genotype of the fetal cells might undergo changes as the cells go through repeated cycles of cell division during the culture step.

Other methods involve the use of antibodies that bind to fetal cells. The antibodies can be immobilised (Mueller et al., 1990, "Isolation of fetal tropoblasts cells from peripheral blood of pregnant women" The Lancet 336: 197-200) or labeled with binding moieties which allow sorting of labeled and unlabelled cells (e.g. Herzenberg et al., 1979 "Fetal cells in the blood of pregnant women: detection and enrichment by fluorescence-activated cell sorting", Proc. Natl. Acad. Sci (USA) 76:1453-1455 and Little et al. - 1997, Blood, vol 89, No. 7, pp 2347-2358).

WO2005/123779 provides antibodies that preferentially bind to fetal cells rather than material cells and discloses methods of using such antibodies to isolate fetal cells.

The cluster differentiation (CD) antigens are a group of markers which are commonly targeted with specific monoclonal antibodies to isolate cells which carry a particular CD antigen (e.g. Bianchi et al., 1996 Proc. Natl. Acad. Sci (USA) 93: 705-708). This method involves incubation of the cells with a suitable monoclonal antibody labeled e.g. with a fluorescent dye or paramagnetic beads, followed by separation of antibody-bound cells from free cells using fluorescence-activated cell sorting or magnetically activated cell sorting technology respectively. The results obtained in these studies were unsatisfactory, not yielding a sufficient proportion of fetal cells for reliable analysis. This is because to date, no suitable cell marker which is unique to fetal cells and could be targeted with antibodies has been identified. The antibodies that have been tried to date, including those to CD antigens, target both fetal and maternal cells.

The ratios of maternal cells to fetal cells obtained with these antibodies are unsatisfactory, with samples containing such low proportions of fetal cells that they are not suitable for reliable analysis.

There is thus a need for a method of obtaining a sample that contains an analysable concentration of fetal cells. The present inventors have unexpectedly found that by isolating the CD34⁺ population of cells from maternal blood and selecting those cells which are capable of adhering to a solid support, a significant and clinically useful proportion of fetal cells can be obtained. The solid support should preferably be transparent and have good optical properties for microscopic inspection. The solid support has or consists of a glass or plastic surface. Preferably, the solid support is tissue culture grade plastic. Suitable culture grade plastic vessels are those manufactured by Corning Incorporated, New York, USA. The solid support may be coated with a suitable substance such as protein, e.g. collagen or fibronectin, glycosaminoglycans, e.g. hapran sulphate or hyaluronic acid or complex carbohydrates, only coatings which do not significantly encourage non-fetal cells from binding are contemplated by the present invention.

The fetal cells which are enriched according to this method are preferably mononuclear, small (approximate diameter of 10 microns) with lymphocyte-like morphology and having a high nucleus: cytoplasm ratio.

Thus in one aspect, there is provided a method of enriching fetal cells in a maternal blood sample wherein cells which are CD34⁺ and capable of adhering to a solid support are selected, said method comprising, in any order:
a) enrichment of the sample for CD34⁺ cells; and
b) contacting the sample with a solid support and harvesting the cells which adhere to said solid support by harvesting cells able to resist vigorous washing without detaching from the support, wherein said solid support has or consists of a glass or plastic surface.

Alternatively viewed, the invention provides a method of preparing a cell sample which is enriched for fetal cells which method comprises subjecting a maternal blood sample to a procedure which selects those cells which are CD34⁺ and capable of adhering to a solid support said method comprising, in any order:
a) enrichment of the sample for CD34⁺ cells; and
b) contacting the sample with a solid support and harvesting the cells which adhere to said solid support by harvesting cells able to resist vigorous washing without detaching from the support, wherein said solid support has or consists of a glass or plastic surface.

Alternatively viewed, the invention provides a method of obtaining an analysable sample of fetal cells from a maternal blood sample, said method comprising, in any order:
a) enrichment of the sample for CD34⁺ cells; and
b) contacting the sample with a solid support and harvesting the cells which adhere to said solid support by harvesting cells able to resist vigorous washing without detaching from the support, wherein said solid support has or consists of a glass or plastic surface.

These two steps may be performed in either order, preferably step b) is performed on the product of step (a) and thus reference to 'the sample' in step b) and generally throughout the specification (unless otherwise clear from the context) includes reference to a fraction of the starting sample which has already been subjected to an enrichment or other step, typically to select CD34⁺ cells.

'Enrichment' of a sample for cells of a certain type or having certain properties refers to an increase in the concentration and/or the proportion of cells of that type relative to starting levels in the sample. In the present context enrichment preferably refers to an increase in the proportion of target cells relative to non-target cells in the sample before and after the enrichment process step(s). Clearly enrichment may be performed by a step which removes non-target cells or other components from the sample, i.e. by way of negative selection, or more preferably a positive selection step is performed which targets the fetal cells of interest.

The method of the invention is preferably carried but by first obtaining cells which are CD34⁺ and then selecting an adherent sub-population thereof. However, methods in which non-adherent cells are removed first and a subpopulation of the adherent cells which is CD34⁺ is subsequently selected are also contemplated by the inventor.

Methods of enrichment for cells exhibiting particular cell surface markers such as CD34 are well known in the art. Methods will preferably involve an affinity ligand to CD34.

The affinity ligand is preferably an antibody. Both polyclonal and monoclonal antibodies can be used, monoclonal antibodies being preferred. The affinity ligand must bind, attach to or complex with the target marker (typically CD34 or a marker which is co-expressed with CD34) in a manner that ensures that the ligand and the cell possessing the target marker can be separated from ligand-free cells.

Preferably, a selectable label which can bind to the antibody is contacted with the sample prior to, simultaneously with, or after contacting the sample with the antibody. The label can consist e.g. of magnetic beads or a fluorescent dye.

Conveniently, the antibodies may be conjugated with markers, such as magnetic beads, which allow for direct separation, biotin, which can be removed with avidin or streptavidin bound to a support, fluorochromes, which can be used with a fluorescence activated cell sorter, or the like, to allow for ease of separation of the particular cell type. Preferably the technique employed is not unduly detrimental to the integrity of the cells, it being preferred that the majority of the intracellular components, in particular the nucleic acid, remain within the cells.

Procedures for separation may include magnetic separation if magnetic beads are used, affinity chromatography, cytotoxic agents joined to the antibody or used in conjunction with said antibody, e.g., complement and cytotoxins, and "panning" with antibody attached to a solid matrix, e.g., plate, or other convenient technique. Techniques providing accurate separation include fluorescence activated cell sorters, which can have varying degrees of sophistication, e.g., a plurality of colour channels, low angle and obtuse light scattering detecting channels, impedance channels, etc.

The methods of the invention result in enrichment of cells which are capable of adhering to certain solid supports, clearly one simple way of ensuring that the cells have this capability is by selecting the sub-population which is in fact adherent. Adherence, can be defined as being able to resist vigorous washing without detaching from the solid support. Vigorous washing may comprise immersion with manual rinsing actions. Some cells inevitably may be lost in the washing step but in general ability to adhere through this washing step will result in significant enrichment of fetal cells.

In a preferred embodiment, adherent cells are selected in the following manner. The cell sample is contacted with the solid support and incubated for a minimum of 1 or 2 hours and for up to 18 hours, preferably for about 2-3 hours at a temperature range of 20-38°C, preferably 35-38°C, even more preferably at 37° C. Most preferably, the incubation is carried out at 37° for about 2 hours. Cells which adhere to the solid support are then harvested by washing off any non-adherent cells. The washing step can be carried out with any suitable buffer or cell culture medium. A preferred wash solution is HBSS (Hanks Balanced salt solution) but other suitable media are known to the person skilled in the art. Preferably, the solution is water based and isotonic. The washing step simply involves immersion of the solid support in the wash solution followed by one or more thorough rinsing actions. Preferably, at least two, more preferably three vigorous washes are performed to remove as many of the non-adherent cells as possible.

The sample on which the method of the invention is performed is usually a sample of peripheral blood from a pregnant mammal. The method of the invention can be used on its own or in combination with other procedures. The term "maternal blood sample" encompasses thus a variety of samples, including a sample which has been taken from the maternal blood stream without further modification and a sample which has undergone one or more processing steps. Preferably, the sample consists of peripheral blood, i.e. blood found in the circulation vasculature. More preferably, the sample has been processed to remove unwanted non-cellular material from the sample. Thus "blood sample" also encompasses fractions and partially purified parts of total blood, in particular fractions which are enriched for dendritic cells or lymphocytes.

In a preferred embodiment the methods of the invention are carried out on the mononuclear faction of the maternal blood sample. The skilled man is well aware of way in which to obtain the mononuclear fraction. For example, mononuclear cells can be separated from other components of peripheral blood by centrifugation, preferably density gradient centrifugation and most preferably discontinuous density gradient centrifugation. Preferably the mononuclear fraction of the blood sample is separated using a Lymphoprep^{™} (Axis Shield) density gradient. Thus preferred methods of the invention comprise a preliminary step in which the sample is enriched for mononuclear cells. The mononuclear fraction will include fetal cells as well as the maternal lymphocytes. Mature maternal red blood cells are of higher density and will be entirely or largely excluded.

The method of the present invention can be carried out on a blood sample from any pregnant mammal. Preferably, the mammal is a human, equine or bovine mammal, most preferably a human. The maternal blood sample may be taken at any time after 7 weeks during pregnancy, preferably between weeks 10 and 20. The blood sample may be as little as a finger prick droplet, but is preferably at least 1 ml, more preferably at least 5 ml, e.g. 10 ml or 20 ml, but higher volumes are also suitable. The method of the invention is preferably carried out on fresh samples, i.e. within 45-75 min e.g. 1 hour after collection of the blood sample. However, processing may also be delayed until several hours after collection. The sample is preferably stored at 4ºC and can be stored conveniently in this way for up to 18 hours.

Once a sample of cells has been obtained, this may be dried and fixed on a solid support and stored at - 20ºC for up to several weeks.

By using the method of the present invention, the inventor was able to obtain samples containing up to 10-30% fetal cells. This level of enrichment of fetal cells has not previously been described. Disclosed herein is an analysable sample of fetal cells obtained from peripheral maternal blood comprising at least 10% of fetal cells. Preferably, the sample comprises at least 15 or 20% of fetal cells, e.g. 10-30%, more preferably at least 25% or 30%. Disclosed herein is a cell sample which comprises 10-30% fetal cells and 90-70% maternal cells. It being appreciated that such a cell sample has a single source and is not provided simply by placing in admixture a maternal and a fetal cell sample.

Also disclosed is a kit for use in the methods of the invention comprising
a) an affinity ligand to CD³⁴; and
b) a solid support

Optionally, the kit also comprises a suitable wash solution such as a buffer, e.g. HBSS. Suitable ligands and supports are described above.

The term "selection" as used herein should be understood to mean either positive or negative selection. Positive selection refers to retaining or collecting the desired cell population and negative selection refers to removing the undesired cell population.

By "analysable sample" is meant a sample which contains a sufficient number of fetal cells to allow direct analysis of the fetal cells such as, for example, by FISH analysis. Preferably, the number and proportion of fetal cells in the analysable sample is sufficiently high to give clinically acceptable success rates.

"CD34⁺ cells" are those cells which express the cluster differentiation antigen 34 and thus have the CD34 glycoprotein on their cell surface. A CD34⁺ cell population or fraction is to be understood as referring to a population of cells which are predominantly CD34⁺. Preferably, the CD34⁺ population is substantially free of any CD34⁻ cells, e.g. comprising at least 80% CD34⁺ cells, more preferably at least 90% CD34⁺ cells, most preferably at least 95% or 98% CD34⁺ cells.

The method of the present invention is particularly suited to obtain an analysable sample of fetal cells for non-invasive fetal diagnosis and gender determination. Disclosed herein is a method of fetal gender determination which method comprises subjecting a sample of maternal blood enriched for fetal cells as described above, and preferably obtained according to one of the methods described above, to a procedure which enables male and female cells to be distinguished. The cells will typically be distinguished at the chromosomal level, XX being differentiated from XY, e.g., by FISH.

Also disclosed is a method of diagnosing a fetal genetic abnormality (i.e. any condition, whether inherited or not, for which undesirable phenotypic characteristics can be linked to a particular genotype), which method comprises contacting a sample of maternal blood enriched for fetal cells as described above, and preferably obtained according to one of the methods described above with an agent capable of distinguishing between genetically normal and genetically abnormal cells.

In a further aspect there is provided a method of enriching fetal cells in a maternal blood sample wherein cells which are CD34⁺ and capable of adhering to a solid support are selected as described herein, wherein said selected cells are subjected to genetic analysis. By "genetic analysis" is meant any analysis of the genetic material of the cells, including analysis of the number, shape and size of chromosomes, presence or absence of specific genes or nucleic acid sequences, and screening for any kinds of mutations such as deletions, insertions, point mutations and the like. Preferably, said genetic analysis includes screening for a fetal genetic abnormality. Said genetic analysis may also or alternatively include gender determination.

The sample of fetal cells that can be obtained with the method of the present invention is suitable for both direct and indirect analysis. Examples of methods of analysis which can be carried out on the samples obtainable by the method of the invention or more generally in conjuction with the methods of the present invention are given below but it should be understood that the skilled person will be aware of other methods of analysis which can be carried out on the samples of the invention. Direct methods of analysis are preferred and include FISH (fluorescence in situ hybridisation). For example, probes which bind the X and Y chromosomes respectively can be used to determine the gender of the fetus. Multicolor FISH can be used to detect chromosomal abnormalities in fetal cells. Probes specific for each individual chromosome each labelled with a different colour are available and these can be used to analyse the number and shape of the chromosomes present in the fetal cells. Suitable probes are described for example in Griffin, D.K., Handyside, A.H., Harper, J. et al. (1994) Clinical experience with preimplantation diagnosis of sex by dual fluorescent in situ hybridisation. J. Assist. Reprod. Genet., 11, 132-143; Munné, S., Tang, Y.X., Grifo, J. et al. (1994) Sex determination of human embryos using the polymerase chain reaction and confirmation by fluorescence in situ hybridization. Fertil. Steril., 61, 111-117; Staessen, C., Van Assche, E., Joris, H. et al. (1999) Clinical experience of sex determination by fluorescent in situ hybridization for preimplantation genetic diagnosis. Mol. Hum. Reprod., 5, 382-389.

A different method of gender determination which may be used in addition or in the alternative is PCR amplification of the male-specific sry gene. The prior art describes suitable primers, e.g. Erdal and Barlas, Turkish Journal of Medical Science 30, 2000, pages 501-503. Suitable primers are also commercially available, e.g. from Fisher Life Science Catalog or Maxim Biotech.

A variety of different probes/visualisation methods may be used to detect the various genetic abnormalities which involve trisomy or large deletions. For Syndromes caused by microdeletions specific probes are preferred.

Suitable methods for carrying out an analysis of the fetal cells are known to the skilled person and are described e.g. in the following references which also detail particular genetic disorders.

Boghosian-Sell L et al. Molecular mapping of the Edwards syndrome phenotype to two noncontiguous regions on chromosome 18. Am J Hum Genet 1994; 476-83.

Helali N et al. A case of duplications 13q32-->qter and deletion of 18p11.32-->pter with mild phenotype: Patau syndrome and duplications of 13q revisited. J Med Genet 1996; 33: 600-2.

Witters I et al. Rapid prenatal diagnosis of trisomy 21 in 5049 consecutive uncultured amniotic fluid samples by fluorescence in situ hybridisation (FISH) Prenat Diagn 2002; 22: 29-33.

Oskarasdottir S et al. Incidence and prevalence of the 22q11 deletion syndrome: a population-based study in Western Sweden. Arch Dis Child 2004; 89: 148-51.

Marinescu RC et al. FISH analysis of terminal deletions in patients diagnosed with cri-du-chat syndrome. Clin Genet 1999; 56: 282-8.

In further applications, a viable enriched sample may be induced to divide and arrest in metaphase of the cell cycle in order to obtain full cytogenic analysis.

Further the enriched samples may be suitable when screening blood to be used for transfusions when nucleated white cell transfusions are required. The samples may also be used when screening dendritic cell and donor lymphocyte infusions as these can also result in graft-versus-host disease. Bone marrow and umbilical cord blood may also be used in transplants to treat haematological deficiency conditions and the same enrichment methods may be performed on such samples in order to perform phenotypic or more preferably genotypic analysis of fetal cells within the sample.

The invention will be further described in the following non-limiting Examples.

### Example 1

Blood samples were taken from peripheral blood of pregnant women at termination of pregnancy and from mothers of sons. The mononuclear cell fraction was separated from the whole sample by density gradient centrifugation through Lymphoprep^{™} and the CD³⁴ positive cell fraction was then separated from the mononuclear cells using MiniMACS technology (Miltenyi Biotech). For this, cells were first labelled with anti-CD³⁴ monoclonal antibody and then with paramagnetic micro beads. The labelled cells were loaded onto a column held in a magnet, the unlabelled cells were eluted and then the labelled cells were released by removing the column from the magnet. The purified CD³⁴ positive cells were incubated on a glass or tissue culture plastic microscope slide at 37°C for at least 2 hours. The non-adherent cells were removed by washing with Hanks' Balanced Salt Solution (HBSS). The slides were air-dried and fixed in methanol: glacial acetic acid for 30 minutes. The slides may then be stored for several weeks prior to analysis by wrapping in foil and freezing at -20°C.

### Fluorescence in situ hybridisation (FISH)

Slides that had been stored frozen were brought to room temperature before they were unwrapped. The X/Y dual label probe was warmed to 37°C. The slides were dehydrated through an ethanol series (75%, 95%, 100% - 1 minute in each) and then air dried. 5µl of the probe was placed in the centre of the cells on the slide. The preparation was covered with a small coverslip which was sealed around the edges with rubber solution. The slide was placed on a hotplate at 73°C for 5 minutes then in a humidified chamber at 37°C overnight. The next morning, the rubber solution and coverslip were removed and the slide mounted in DAPI 4',6-Diamidino-2-phenlyindole antifade solution.

### Analysis of fetal cells in the enrichment sample

The slides were viewed on an Olympus fluorescence microscope attached to a MacIntosh computer. At least 50 cells per sample were scored by the eye. The Y chromosome probe was stained with SpectrumGreen and the X chromosome probe with SpectrumOrange. Thus, male cells will yield one green and one red signal; female cells will have two red signals. In the samples tested, 10-27% of the cells contained the male Y chromosome

### Example 2

Table 1 below lists some chromosomal disorders which can be detected in fetal cells enriched according to the methods of the present invention.

**Table 1**

| **Syndrome** | **Cytogenetic and phenotypic features** |
|---|---|
| Edwards | Trisomy 18; 1/3000 births; mental retardation; dysmorphism; survival < 1 year |
| Patau | Trisomy 13; 1/5000; motor/mental retardation; survival < 6 months |
| Downs | Trisomy 21; mongolism |
| DiGeorge | Deletion 22q11; cardiac malformation; endocrine/immune anomalies; facial attributes |
| Wolf-Hirschom | Deletion 4p; 1/50000 births; midline fusion defects; 34% mortality in 2 years |
| Cri du chat | Deletion 5p; mental handicap; characteristic cry Various chromosomes; dysmorphic features |

A variety of different probes/visualisation methods may be used to detect the various conditions which involve trisomy or large deletions. For Syndromes caused by microdeletions specific probes are required.

Suitable methods for carrying out an analysis of the fetal cells are known to the skilled person and are described e.g. in the following references which also detail particular genetic disorders.

Boghosian-Sell L et al. Molecular mapping of the Edwards syndrome phenotype to two noncontiguous regions on chromosome 18. Am J Hum Genet 1994; 476-83.

Helali N et al. A case of duplications 13q32-->qter and deletion of 18p11.32-->pter with mild phenotype: Patau syndrome and duplications of 13q revisited. J Med Genet 1996; 33: 600-2.

Witters I et al. Rapid prenatal diagnosis of trisomy 21 in 5049 consecutive uncultured amniotic fluid samples by fluorescence in situ hybridisation (FISH) Prenat Diagn 2002; 22: 29-33.

Oskarasdottir S et al. Incidence and prevalence of the 22q11 deletion syndrome: a population-based study in Western Sweden. Arch Dis Child 2004; 89: 148-51.

Marinescu RC et al. FISH analysis of terminal deletions in patients diagnosed with cri-du-chat syndrome. Clin Genet 1999; 56: 282-8.

## Claims

1. A method of enriching fetal cells in a maternal blood sample wherein cells which are CD34⁺ and capable of adhering to a solid support are selected, said method comprising, in any order:
a) enrichment of the sample for CD34⁺ cells; and
b) contacting the sample with a solid support and harvesting the cells which adhere to said solid support by harvesting cells able to resist vigorous washing without detaching from the support, wherein said solid support has or consists of a glass or plastic surface.

2. A method according to claim 1, wherein step (b) is performed on the product of step (a).

3. A method according to claim 1, wherein step (a) is performed on the product of step (b).

4. A method according to any one of claims 1-3 wherein an affinity ligand to CD34 is used.

5. A method according to claim 4 wherein said affinity ligand is an antibody.

6. A method according to claim 4 or 5 wherein a selectable label which can bind to said affinity ligand is contacted with the sample prior to, simultaneously with, or after contacting the sample with said affinity ligand.

7. A method according to claim 6, wherein said selectable label consist of magnetic beads or a fluorescent dye.

8. A method according to any one of claims 4 to 7 wherein said affinity ligand is conjugated with a marker such as magnetic beads, biotin or fluorochromes.

9. A method according to any one of claims 1-8 wherein selection of adherent cells comprises contacting the sample with a solid support and incubating for 1 to 18 hours at a temperature range of 20-38ºC.

10. A method according to any one of claims 1-9 wherein the method is carried out on the mononuclear faction of the maternal blood sample.

11. A method according to any one of claims 1-10, wherein said selected cells are subjected to genetic analysis.

12. A method according to claim 11, wherein said genetic analysis includes gender determination.

13. A method according to claim 11 or 12, wherein said genetic analysis includes screening for a fetal genetic abnormality.

## Patentansprüche

1. Verfahren zur Anreicherung von Fötalzellen in einer mütterlichen Blutprobe, bei dem Zellen, bei denen es sich um CD34⁺-Zellen handelt und die auf einem festen Träger haften können, ausgewählt werden, wobei das Verfahren folgende Schritte in beliebiger Reihenfolge umfasst:
a) Anreicherung der Probe auf CD34⁺-Zellen; und
b) Inberührungbringen der Probe mit einem festen Träger und Abernten der Zellen, die an dem festen Träger haften, durch Abernten von Zellen, die kräftigem Waschen widerstehen können, ohne sich von dem Träger abzulösen, worin der feste Träger eine Glas- oder Kunststoffoberfläche aufweist oder daraus besteht.

2. Verfahren nach Anspruch 1, worin Schritt (b) an dem Produkt aus Schritt (a) durchgeführt wird.

3. Verfahren nach Anspruch 1, worin Schritt (a) an dem Produkt aus Schritt (b) durchgeführt wird.

4. Verfahren nach einem der Ansprüche 1-3, worin ein Affinitätsligand zu CD34 verwendet wird.

5. Verfahren nach Anspruch 4, worin der Affinitätsligand ein Antikörper ist.

6. Verfahren nach Anspruch 4 oder 5, worin eine auswählbare Markierung, die an den Affinitätsliganden binden kann, vor, gleichzeitig mit oder nach dem Inberührungbringen der Probe mit dem Affinitätsliganden mit der Probe in Berührung gebracht wird.

7. Verfahren nach Anspruch 6, worin die auswählbare Markierung aus Magnetperlen oder einem fluoreszierenden Farbstoff besteht.

8. Verfahren nach einem der Ansprüche 4-7, worin der Affinitätsligand mit einer Markierung, wie Magnetperlen, Biotin oder Fluorchrom, konjugiert ist.

9. Verfahren nach einem der Ansprüche 1-8, worin bei der Auswahl haftender Zellen die Probe mit einem festen Träger in Berührung gebracht wird und 1 bis 18 Stunden bei einer Temperatur im Bereich von 20-38°C inkubiert wird.

10. Verfahren nach einem der Ansprüche 1-9, worin das Verfahren an dem mononukleären Teil der mütterlichen Blutprobe durchgeführt wird.

11. Verfahren nach einem der Ansprüche 1-10, worin die ausgewählten Zellen einer Genanalyse unterworfen werden.

12. Verfahren nach Anspruch 11, worin die Genanalyse eine Geschlechtsbestimmung beinhaltet.

13. Verfahren nach Anspruch 11 oder 12, worin die Genanalyse ein Screening auf fötale Genanomalien beinhaltet.

## Revendications

1. Procédé d'enrichissement de cellules foetales dans un échantillon de sang maternel dans lequel des cellules qui sont CD34⁺ et capables d'adhérer à un support solide sont sélectionnées, ledit procédé comprenant, dans n'importe quel ordre :
a) l'enrichissement de l'échantillon en cellules CD34⁺ ; et
b) la mise en contact de l'échantillon avec un support solide et le recueil des cellules qui adhèrent audit support solide par recueil de cellules aptes à résister à un lavage vigoureux sans se détacher du support, ledit support solide ayant ou étant constitué d'une surface en verre ou en matière plastique.

2. Procédé selon la revendication 1, dans lequel l'étape (b) est effectuée sur le produit de l'étape (a).

3. Procédé selon la revendication 1, dans lequel l'étape (a) est effectuée sur le produit de l'étape (b) .

4. Procédé selon l'une quelconque des revendications 1-3 dans lequel un ligand par affinité pour CD34 est utilisé.

5. Procédé selon la revendication 4 dans lequel ledit ligand par affinité est un anticorps.

6. Procédé selon la revendication 4 ou 5 dans lequel un marqueur de sélection qui peut se lier audit ligand par affinité est mis en contact avec l'échantillon avant, simultanément avec ou après la mise en contact de l'échantillon avec ledit ligand par affinité.

7. Procédé selon la revendication 6, dans lequel ledit marqueur de sélection consiste en des billes magnétiques ou un colorant fluorescent.

8. Procédé selon l'une quelconque des revendications 4 à 7 dans lequel ledit ligand par affinité est conjugué avec un marqueur tel que des billes magnétiques, la biotine ou des fluorochromes.

9. Procédé selon l'une quelconque des revendications 1-8 dans lequel la sélection de cellules adhérentes comprend la mise en contact de l'échantillon avec un support solide et l'incubation pendant 1 à 18 heures à une plage de température de 20-38 °C.

10. Procédé selon l'une quelconque des revendications 1-9, le procédé étant effectué sur la fraction mononucléaire de l'échantillon de sang maternel.

11. Procédé selon l'une quelconque des revendications 1-10, dans lequel lesdites cellules sélectionnées sont soumises à une analyse génétique.

12. Procédé selon la revendication 11, dans lequel ladite analyse génétique comprend la détermination du sexe.

13. Procédé selon la revendication 11 ou 12, dans lequel ladite analyse génétique comprend le dépistage d'une anomalie génétique foetale.
